# EUROPEAN PATENT APPLICATION

(11) **EP 3 936 611 A1**
(43) Date of publication of application: **12.01.2022**
(21) Application number: 19917821.1
(22) Date of filing: 05.03.2019
(51) Int. Cl.: C12N 5/0783, A61K 35/17, A61P 35/00

(54) **COMPOSITION, CULTURE MEDIUM AND METHOD FOR INDUCING AND/OR AMPLIFYING TSCM IN VITRO**

(71) Applicant: Shanghai Sinobay Biotechnology Co., Ltd., Shanghai 201506 (CN)
(72) Inventor: XU, Jianqing, Shanghai 201506 (CN); ZHANG, Xiaoyan, Shanghai 201506 (CN); LIAO, Qibin, Shanghai 201506 (CN)
(74) Representative: Uexküll & Stolberg
(86) International application number: PCT/CN2019/076957
(87) International publication number: WO 2020/177071

(57) **Abstract**

A composition for inducing and/or amplifying T_{SCM} in vitro, a culture medium including the composition, and a method for inducing and/or amplifying T_{SCM} in vitro are provided, wherein the composition comprises inducing agents including IL-7 and IL-21. The chimeric antigen receptor T-memory stem cells induced differentiated and amplified by adding the composition can be used directly for reinfusion therapy of patients.

## Description

### Technical Field

The present invention belongs to the field of cell culture. In particular, the present invention relates to a composition, culture medium and method for inducing and/or amplifying cells. More particularly, the present invention relates to a composition, culture medium and method for inducing and/or amplifying T-memory stem cells in vitro.

### Background Art

T cells, with strong anti-infection and anti-tumor abilities, are the backbone force of the body's adaptive immune system to defend against pathogens, tumor cells and other foreign bodies. They can not only quickly respond to foreign bodies or body neoplasms, but also provide relatively long-term immune protection memory. Studies have shown that by transfusing memory T cells that are induced and amplified in vitro back into patients' bodies, a variety of diseases, especially cancer that are serious threats to human health, can be treated.

At present, among in-vitro induction and amplification methods of memory T cells, the more mature method is to combine an anti-CD3 antibody, an anti-CD28 antibody, and an IL-2 for stimulating induction and differentiation, and by this method, the proliferation of T cells subjected to stimulated differentiation is significantly accelerated, the number of naive T cells is greatly reduced, while the number of terminally differentiated T cells is significantly increased. Such T cells have a short survival time in vivo, cannot provide a long-term immune protection, and cannot meet the needs of clinical treatment.

In recent years, a new T cell subset has been identified and named T-memory stem cell (T_{SCM}). T_{SCM}, which is in the upstream of memory T cells, has the characteristics of stem cells, has a stronger multi-directional differentiation potential, and can differentiate into central memory T cells (T_{CM}), effector memory T cells (T_{EM}), and effector T cells (T_{EF}), thus producing a large number of effector molecules such as IFN-γ which have a stronger killing ability. Moreover, T_{SCM} also has a self-renewal ability while having the multi-directional differentiation potential, thereby maintaining its homeostasis in the absence of antigens. Studies have shown that T_{SCM} has a stronger anti-tumor ability, and its in-vivo self-renewal ability, secondary immune response intensity and in-vivo survival time are superior to those of traditional memory T cells.

The adoptive reinfusion of an immune cell therapy has encountered a bottleneck problem in clinical practice, and especially a recently rapidly developed chimeric antigen receptor T cell (CAR-T) immunotherapy is facing the problems of how to complete induction and amplification culture of sufficient immune cells within a short time (14 days), and maintain the activity of immune cells as much as possible and maintain "young" immune cells. At present, however, most of the T cells cultured by induction and amplification are terminally differentiated cells, and especially T_{SCM}, which is obtained by the commonly used culture method of combining an anti-CD3 antibody, an anti-CD28 antibody, and an IL-2, has low percentage (only 9 to 20%). First, such cells are difficult to survive for a long time under the conditions of in-vitro culture, and thus it is difficult to obtain a sufficient amount of immune cells; and second, long-term immune protection is difficult to be obtained even if such immune cells are transfused back into patients' bodies. However, the multi-directional differentiation potential and self-renewal ability of T_{SCM} have solved the problems of difficulties in in-vitro induction and amplification and poor in-vivo efficacy of the adoptive reinfusion of the immune cell therapy in clinical practice. Studies have shown that the percentage of T_{SCM}-like CD8⁺CD45RA⁺CCR7⁺CAR-T cells that are induced, amplified and cultured in vitro is positively correlated with the clinical efficacy of tumor immunotherapy, which indicates that T_{SCM} has a stronger in-vivo anti-tumor ability. The reason may be that the strong viability of T_{SCM} greatly prolongs the in-vivo survival time of transfused CAR-T cells. Thus, the strong anti-tumor ability makes T_{SCM} the most effective cell subset in immunotherapy. Therefore, immunotherapy using antigen-specific T_{SCM}, TCR-T_{SCM} or CAR-T_{SCM} that are induced and amplified in vitro will become a new approach and new means of cell therapy.

Based on the above objectives, it is of great practical significance to develop and improve the technical methods for inducting and amplifying T_{SCM} in vitro on purpose and target and to find economical, convenient cytokines or drugs with slight side effects. However, there is still a lack of a convenient and efficient method for massively inducting and amplifying T_{SCM} in vitro in the prior art.

### Summary of the Invention

The aim of the present invention is to provide a composition and culture medium for inducing and/or amplifying T-memory stem cells in vitro to overcome the shortcomings of the prior art. The present invention further provides a method for inducing and/or amplifying T-memory stem cells in vitro by using the composition provided by the present invention. In accordance with the method of the present invention, the proportion of CAR-T_{SCM} in CAR-T cells is significantly increased and the CAR-T_{SCM} can be used directly for a reinfusion therapy of a CAR-T therapy for a patient.

In one aspect, the present invention provides a composition for inducing and/or amplifying T-memory stem cells (T_{SCM}) in vitro. The composition consists of inducing agents comprising interleukin-7 (IL-7) and interleukin-21 (IL-21).

In accordance with the composition of the present invention, the inducing agents further comprise one or more of IL-3, IL-12, IL-15 and IL-18; and preferably, the inducing agents further comprise IL-15.
In accordance with the composition of the present invention, the inducing agents further comprise a GSK-3β inhibitor; preferably, the GSK-3β inhibitor is TWS119;
In accordance with the composition of the present invention, the inducing agents comprise IL-7, IL-21, IL-15 and a GSK-3β inhibitor; and
in accordance with the composition of the present invention, the inducing agents are added to a culture medium, coupled to magnetic beads, coated on a cell culture plate, or expressed on cells in the form of free proteins.

In accordance with the composition of the present invention, working concentrations of various inducing agents are as follows:
a working concentration of each of inducing agents, excluding a GSK-3β inhibitor, ranges from 1 to 100 ng/mL, and a working concentration of a GSK-3β inhibitor ranges from 1 to 50 µM.

In accordance with the composition of the present invention, the working concentrations of various inducing agents in the composition are as follows:
1 to 100 ng/mL of IL-7, and 1 to 100 ng/mL of IL-21;
preferably, the composition further comprises 1 to 100 ng/mL of IL-15; and
preferably, the composition further comprises 1 to 50 µM of a GSK-3β inhibitor.

In another aspect, the present invention provides a culture medium for inducing and/or amplifying T-memory stem cells (T_{SCM}) in vitro, wherein the culture medium comprises a T cell growth basal culture medium and the composition.

In accordance with the culture medium for inducing and/or amplifying T_{SCM} in vitro or a preparation method thereof of the present invention, the method comprises a step of adding the composition into the T cell growth basal culture medium;
preferably, the above step is completed in a manner comprising the steps of:
adding one or more components of the composition in the form of prepared free protein molecules into the T cell growth basal culture medium;
coating one or more components of the composition on magnetic beads and adding the magnetic beads to the T cell growth basal culture medium;
adding feeder cells which are infected or transfected with genetic materials encoding one or more components of the composition in the form of a vector into the T cell growth basal culture medium, and co-culturing the feeder cells and cultured objective cells;
infecting or transfecting the objective cells with genetic materials encoding one or more components of the composition in the form of a vector, and culturing the objective cells with the T cell growth basal culture medium; or
expressing one or more components of the composition by using exogenous cells to allow the exogenous cells to act in a cell supernatant cultured by using the T cell growth basal culture medium.

Preferably, the culture medium may be prepared by using one or a combination of the method.

The present invention further provides use of the composition and/or culture medium for inducing and/or amplifying T_{SCM} in vitro.

In accordance with the use of the present invention, the T_{SCM} is a peripheral blood mononuclear cell (PBMC), a CD4⁺T cell, a CD8⁺T cell or a CD4⁻CD8⁻T cell.

In accordance with the use of the present invention, the T_{SCM} is a CAR-T cell, a TCR-T cell or a tumor infiltrating lymphocyte.

In still another aspect, the present invention provides a method for inducing and/or amplifying T_{SCM} in vitro, which comprises the step of inducing and/or amplifying cells by using the composition and/or culture medium of the present invention;
in accordance with the the method for inducing and/or amplifying T_{SCM} in vitro of the present invention comprises the steps of:
   ① separating peripheral blood mononuclear cells (PBMCs), CD4⁺T cells, CD8⁺T cells or CD4⁻CD8⁻T cells;
   ② placing the cells obtained in step ① in the culture medium of the present invention or in a T cell growth basal culture medium while adding the composition, adding a stimulant, and culturing for 6 to 7 days,
preferably, the stimulant is one or more of an anti-CD3 antibody, an anti-CD28 antibody/CD28 ligand, an anti-CD137 antibody/CD137 ligand, an anti-OX40 antibody/OX40 ligand, an anti-CD160 antibody/CD160 ligand, a Toll-like receptor 1 (TLR1) ligand, a Toll-like receptor 2 (TLR2) ligand, a Toll-like receptor 5 (TLR5) ligand, a Toll-like receptor 6 (TLR6) ligand, a retinoic acid-induced gene protein (RIG-I) ligand, a chimeric antigen receptor target antigen, a PD-1 blocking antibody, a CTLA-4 blocking antibody, an LAG-3 blocking antibody, a Tm-3 blocking antibody, and a BTLA blocking antibody, and more preferably, the stimulant is magnetic beads coupled to an anti-CD3 antibody and an anti-CD28 antibody; and
③ placing the cells obtained in step ② in the culture medium of the present invention or in the T cell growth basal culture medium while adding the composition, supplementing the culture medium of the present disclosure or the T cell growth basal culture medium while adding the composition every 2 to 4 days until the end of a cell culture cycle.

In a preferred embodiment, the method comprises the steps of:
① selecting CD8⁺T cells by a magnetic bead negative selection method;
② placing the cells obtained in step ① in the T cell growth basal culture medium, adding the composition at the working concentration, adding magnetic beads coupled to an anti-CD3 antibody and an anti-CD28 antibody, and culturing for 7 days; and
③ placing the cells obtained in step ② in the culture medium of the present invention or in T cell growth basal culture medium while adding the composition, supplementing a fresh culture medium or a fresh T cell growth basal culture medium while adding the composition every 3 days from Day 4 until the end of a cell culture cycle.

In a preferred embodiment, the method is a method for inducing and/or amplifying chimeric antigen receptor T-memory stem cells (CAR-T_{SCM}) in vitro, which comprises the steps of:
1) separating peripheral blood mononuclear cells (PBMCs), CD4⁺T cells, CD8⁺T cells or CD4⁻CD8⁻T cells;
2) placing the cells obtained in step 1) in the culture medium of the present invention or in the T cell growth basal culture medium while adding the composition, adding a stimulant, and culturing for 1 to 2 days, preferably, the stimulant is one or more of an anti-CD3 antibody, an anti-CD28 antibody/CD28 ligand, an anti-CD137 antibody/CD137 ligand, an anti-OX40 antibody/OX40 ligand, an anti-CD160 antibody/CD160 ligand, a Toll-like receptor 1 (TLR1) ligand, a Toll-like receptor 2 (TLR2) ligand, a Toll-like receptor 5 (TLR5) ligand, a Toll-like receptor 6 (TLR6) ligand, a retinoic acid-induced gene protein (RIG-I) ligand, a chimeric antigen receptor target antigen, a PD-1 blocking antibody, a CTLA-4 blocking antibody, an LAG-3 blocking antibody, a Tm-3 blocking antibody, and a BTLA blocking antibody, and
   more preferably, the stimulant is magnetic beads coupled to an anti-CD3 antibody and an anti-CD28 antibody;
3) transfecting or infecting the cells obtained in step 2) with a vector carrying a chimeric antigen receptor; and
4) placing the cells obtained in step 3) in the culture medium of the present invention or in the T cell growth basal culture medium while adding the composition, adding a stimulant, continuing culturing for 5 to 6 days, and then removing the stimulant; supplementing the culture medium or the T cell growth basal culture medium while adding the composition every 2 to 4 days from Day 4 until the end of a culture cycle of CAR-T cell,

preferably, the stimulant is one or more of an anti-CD3 antibody, an anti-CD28 antibody/CD28 ligand, an anti-CD137 antibody/CD137 ligand, an anti-OX40 antibody/OX40 ligand, an anti-CD160 antibody/CD160 ligand, a Toll-like receptor 1 (TLR1) ligand, a Toll-like receptor 2 (TLR2) ligand, a Toll-like receptor 5 (TLR5) ligand, a Toll-like receptor 6 (TLR6) ligand, a retinoic acid-induced gene protein (RIG-I) ligand, a chimeric antigen receptor target antigen, a PD-1 blocking antibody, a CTLA-4 blocking antibody, an LAG-3 blocking antibody, a Tm-3 blocking antibody, and a BTLA blocking antibody, and
more preferably, the stimulant is magnetic beads coupled to an anti-CD3 antibody and an anti-CD28 antibody.

In a preferred embodiment, the method comprises the steps of:
1) separating peripheral blood mononuclear cells (PBMCs) or CD8⁺T cells;
2) placing the cells obtained in step 1) in the culture medium of the present invention or in the T cell growth basal culture medium while adding the composition, adding magnetic beads coupled to an anti-CD3 antibody and an anti-CD28 antibody, and culturing for 1 day;
3) adding a lentiviral vector LV-CAR carrying the chimeric antigen receptor into the cells obtained in step 2) in a proportion of multiplicity of infection (MOI) of 2-15, and performing centrifugal infection at 30 to 32°C for 2 h; and
4) after centrifugation is finished, culturing the cells obtained in step 3) overnight, placing the cells in the culture medium of the present invention or in the T cell growth basal culture medium while adding the composition, adding magnetic beads coupled to an anti-CD3 antibody and an anti-CD28 antibody, continuing culturing for 5 to 6 days, and then removing the magnetic beads coupled to an anti-CD3 antibody and an anti-CD28 antibody. Supplementing the culture medium or the T cell growth basal culture medium while adding the composition every 3 days from Day 4, and continuing culturing until the end of a culture cycle of CAR-T cells,

preferably, the stimulant is one or more of an anti-CD3 antibody, an anti-CD28 antibody/CD28 ligand, an anti-CD137 antibody/CD137 ligand, an anti-OX40 antibody/OX40 ligand, an anti-CD160 antibody/CD160 ligand, a Toll-like receptor 1 (TLR1) ligand, a Toll-like receptor 2 (TLR2) ligand, a Toll-like receptor 5 (TLR5) ligand, a Toll-like receptor 6 (TLR6) ligand, a retinoic acid-induced gene protein (RIG-I) ligand, a chimeric antigen receptor target antigen, a PD-1 blocking antibody, a CTLA-4 blocking antibody, an LAG-3 blocking antibody, a Tm-3 blocking antibody, and a BTLA blocking antibody, and
more preferably, the stimulant is magnetic beads coupled to an anti-CD3 antibody and an anti-CD28 antibody.

In a preferred embodiment, the method is a method for inducing and/or amplifying T cell receptor T-memory stem cells (TCR-T_{SCM}) in vitro, which comprises the steps of:
1) separating peripheral blood mononuclear cells (PBMCs), CD4⁺T cells, or CD8⁺T cells;
2) placing the cells obtained in step 1) in the culture medium of the present invention or in the T cell growth basal culture medium while adding the composition, adding a stimulant, and culturing for 1 to 2 days, preferably, the stimulant is one or more of an anti-CD3 antibody, an anti-CD28 antibody/CD28 ligand, an anti-CD137 antibody/CD137 ligand, an anti-OX40 antibody/OX40 ligand, an anti-CD160 antibody/CD160 ligand, a Toll-like receptor 1 (TLR1) ligand, a Toll-like receptor 2 (TLR2) ligand, a Toll-like receptor 5 (TLR5) ligand, a Toll-like receptor 6 (TLR6) ligand, a retinoic acid-induced gene protein (RIG-I) ligand, a chimeric antigen receptor target antigen, a PD-1 blocking antibody, a CTLA-4 blocking antibody, an LAG-3 blocking antibody, a Tm-3 blocking antibody, and a BTLA blocking antibody, and
   more preferably, the stimulant is magnetic beads coupled to an anti-CD3 antibody and an anti-CD28 antibody;
3) transfecting or infecting the cells obtained in step 2) with a vector carrying a T cell receptor; and
4) placing the cells obtained in step 3) in the culture medium of the present invention or in the T cell growth basal culture medium while adding the composition, adding a stimulant, continuing culturing for 5 to 6 days, and then removing the stimulant. Supplementing the culture medium or the T cell growth basal culture medium while adding the composition every 2 to 4 days from Day 4 until the end of a culture cycle of TCR-T cells.

In a preferred embodiment, the method comprises the steps of:
1) separating peripheral blood mononuclear cells (PBMCs), CD4⁺T cells, or CD8⁺T cells;
2) placing the cells obtained in step 1) in the culture medium of the present invention or in the T cell growth basal culture medium while adding the composition, adding magnetic beads coupled to an anti-CD3 antibody and an anti-CD28 antibody, and culturing for 1 day;
3) adding a lentiviral vector LV-TCR carrying a chimeric antigen receptor into the cells obtained in step 2) in a proportion of multiplicity of infection (MOI) of 2-15, and performing centrifugal infection at 30 to 32°C for 2 h; and
4) after centrifugation is finished, culturing the cells obtained in step 3) overnight, placing the cells in the culture medium of the present invention or in the T cell growth basal culture medium while adding the composition, adding magnetic beads coupled to an anti-CD3 antibody and an anti-CD28 antibody, continuing culturing for 5 to 6 days, and then removing the magnetic beads coupled to an anti-CD3 antibody and an anti-CD28 antibody. Supplementing the culture medium or the T cell growth basal culture medium while adding the composition every 3 days from Day 4, and continuing culturing until the end of a culture cycle of TCR-T cells.

In yet another aspect, the present invention provides a T_{SCM} prepared according to the method of the present invention; and
preferably, the T_{SCM} is a CAR-T_{SCM}, a TCR-T_{SCM} or a tumor infiltrating lymphocyte.

The present invention further provides use of the T_{SCM} of the present invention in the manufacture of a medicament for treating tumors.

In still another aspect, the present invention provides a method for cellular immunotherapeutically treating tumors, which comprises using the T_{SCM} of the present invention.

The inventors of the present invention have found that by adding cytokines IL-7, IL-15 and IL-21 during the culture of T cells, the T cells can be efficiently induced and/or amplified to T_{SCM} in vitro, and the proportion of T_{SCM} in the T cells and the number of T_{SCM} are significantly increased. The method disclosed by the present invention can be used for inducing and/or amplifying chimeric antigen receptor T-memory stem cells (CAR-T_{SCM}) in vitro and also for inducing and amplifying T cell receptor T-memory stem cells (TCR-T_{SCM}) in vitro. The composition used in the present invention is safe and readily available; and TCR-T_{SCM} or CAR-T_{SCM} induced and/or amplified by the composition can be used in cellular therapy for tumor patients, which provides a new strategy for cancer immunotherapy and has an important value for development and popularization.

### Brief Description of the Drawings

The embodiments of the present invention will be described below in combination with drawings in detail.

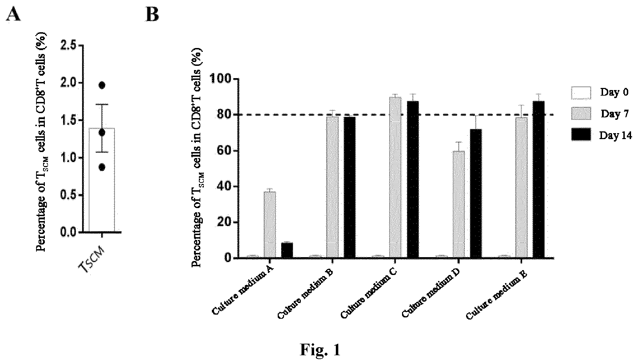

Fig. 1 illustrates an in-vitro induction effect of a culture medium according to the present invention on a CD8⁺T_{SCM}, wherein Fig. 1A illustrates the percentage of T_{SCM} in CD8⁺T cells in healthy human peripheral blood; Fig. 1B is a comparison diagram illustrating effects of inducing CD8⁺T cells to differentiate into T_{SCM} by using the culture medium according to the present invention at Day 7 and Day 14 of culture, and the higher the T_{SCM} proportion, the better the induction effect.

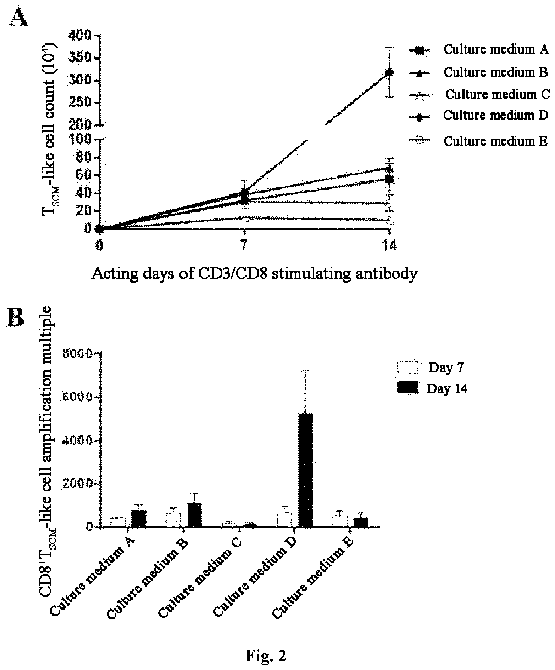

Fig. 2 illustrates an in-vitro induction and amplification effect of the culture medium according to the present invention on the CD8⁺T_{SCM}, wherein Fig. 2A is a cell count diagram illustrating the CD8⁺T_{SCM} induced and amplified by the culture medium according to the present invention. For the same culture time, the more CD8⁺T_{SCM} cells, the better the induction and amplification effects of the composition; and Fig. 2B illustrates induction and amplification multiples of CD8⁺T_{SCM} at Day 7 and Day 14 of culture by using the culture medium according to the present invention.

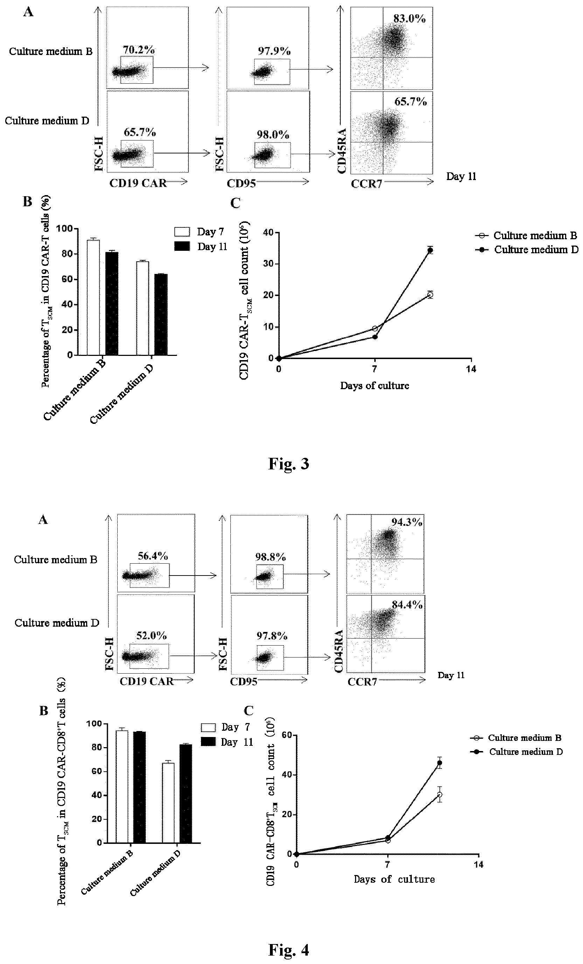

Fig. 3 illustrates in-vitro induced differentiation and amplification effects of two culture media according to the present invention on CD19 CAR-T_{SCM}, wherein Fig. 3A illustrates a result of flow cytometry analysis of T_{SCM} in CD19 chimeric antigen receptor T cells (CD19 CAR-T) on Day 11 of culture; Fig. 3B is a statistical diagram of the percentages of CD19 CAR-T_{SCM} in T cells at Day 7 and Day 11 of culture; and Fig. 3C illustrates in-vitro amplification effects of CD19 CAR-T_{SCM} at Day 7 and Day 11 of culture.
Fig. 4 illustrates in-vitro induced differentiation and amplification effects of two culture media according to the present invention on CD19 CAR-CD8⁺T_{SCM}, wherein Fig. 4A illustrates a result of flow cytometry analysis of T_{SCM} in CD19 CAR-CD8⁺T cells at Day 11 of culture; Fig. 4B is a statistical diagram of the percentages of the CD19 CAR-CD8⁺T_{SCM} in CD8⁺T cells at Day 7 and Day 11 of culture; and Fig. 4C illustrates in-vitro amplification effects of CD19 CAR-CD8⁺T_{SCM} at Day 7 and Day 11 of culture.

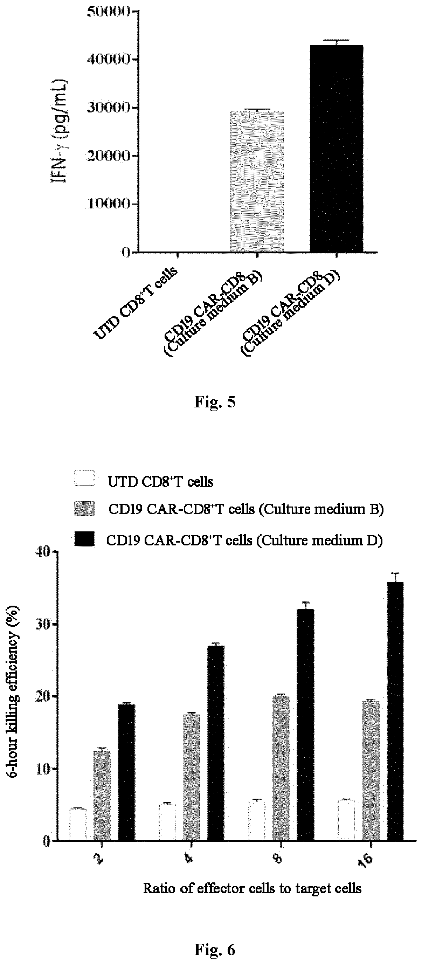

Fig. 5 illustrates an in-vitro activation effect of CD19 CAR-CD8⁺T cells cultured by using two culture media according to the present invention.
Fig. 6 illustrates an in-vitro killing activity of CD19 CAR-CD8⁺T cells cultured by using two culture media according to the present invention.

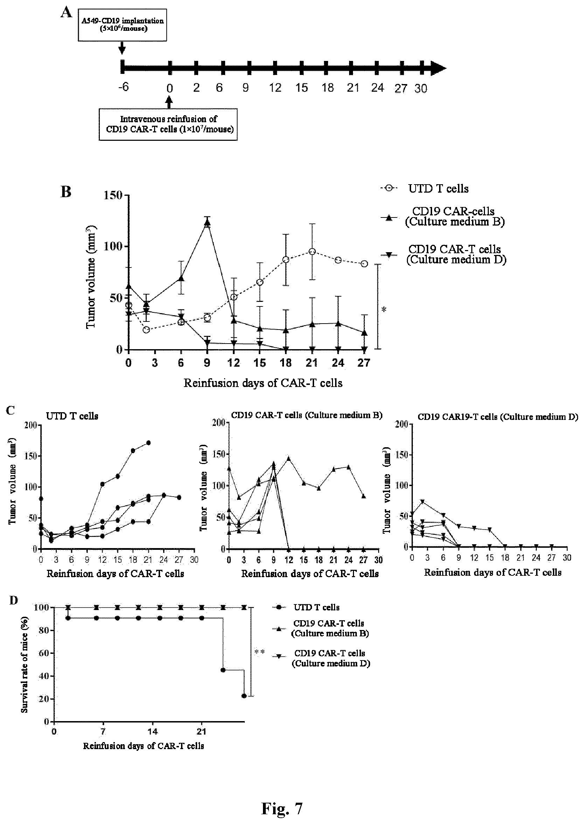

Fig. 7 illustrates an anti-tumor effect of CD19⁺CAR-T cells cultured by using two culture media according to the present invention on severe combined immunodeficiency (NSG) mice.

### Best Mode for Carrying Out the Invention

The following description of the present application is merely an illustration of various embodiments of the present application. Therefore, the specific modifications discussed herein should not be construed as limiting the scope of the application. Multiple equivalents, changes, and modifications can be readily made by those skilled in the art without departing from the scope of the present application, and it should be understood that such equivalent embodiments are to be included within the scope of the present invention. All documents, including publications, patents, and patent applications, cited in the present application, are hereby incorporated by reference in their entirety.

The present invention will now be further described in detail with reference to the accompanying drawings and specific experiments. Unless otherwise indicated, reagents, instruments, devices, and methods used in the present invention are those conventional and commercially available reagents, instruments, devices, and methods used in the art.

Culture media B to E of the present invention were prepared, and a culture medium A was prepared for control, wherein the media contained the following common components:
a T cell growth basal culture medium: a serum-free culture medium for lymphocytes (KBM581, Corning Inc.);
The different components of the media were as follows:
   culture medium A contained 50 ng/mL IL-2;
   culture medium B contained 5 ng/mL IL-7 and 30 ng/mL IL-21;
   culture medium C contained 5 ng/mL IL-7, 30 ng/mL IL-21, and 5 µM TWS119;
   culture medium D contained 5 ng/mL IL-7, 30 ng/mL IL-21 and 10 ng/mL IL-15; and
   culture medium E contained 5 ng/mL IL-7, 30 ng/mL IL-21, 10 ng/mL IL-15, and 5µM TWS119.

### Example 1 In-vitro induction and amplification of CD8⁺T_{SCM} in the culture media of the present invention

1) Pre-prepared human primary T cells (Shanghai Sinobay Bio-Tech Co., Ltd., hereinafter referred to as "Sinobay") were selected by a magnetic bead negative selection method to obtain human primary CD8⁺T cells (CD3⁺CD8⁺), and the human primary CD8⁺T cells (CD3⁺CD8⁺) were placed in a 96-well U-bottom culture plate, with 5×10⁴ CD8⁺T cells in each well;
2) subsequently, culture media A to E and magnetic beads coupled to an anti-CD3 antibody and an anti-CD28 antibody were added into the culture plates containing the CD8⁺T cells, wherein a ratio of the number of the magnetic beads to the number of the cells was 1: 1; and fresh culture media A to E were supplemented at Day 4, Day 7, Day 10 and Day 13 of cell culture; and
3) the cells were counted at Day 0, Day 7 and Day 14 of cell culture respectively, flow cytometry antibodies (CD45RA, CCR7 and CD95) for detecting T_{SCM} were incubated, and the T_{SCM} was detected by using a flow cytometry.

Experimental results are shown in Fig. 1 and Fig. 2.

Fig. 1 is a comparison diagram illustrating in-vitro induction effects of the culture media A to E on CD8⁺T_{SCM}. Fig. 1A illustrates the percentage of T_{SCM} in CD8⁺T cells (namely, primary cells) in healthy human peripheral blood. As shown in Fig. 1A, the percentage of T_{SCM} in primary CD8⁺T cells was very low, which is less than 2%, and Fig. 1B is a comparison diagram illustrating effects of inducing CD8⁺T cells to differentiate into T_{SCM} by using the culture media A to E at Day 7 and Day 14 of culture. Compared with commonly used IL-2, other media B to E could effectively induce CD8⁺T cells to differentiate into T_{SCM}, and had much better induction effects than IL-2. The medium A had an average induction efficiency of less than 10% at Day 14 of culture. However, at Day 14 of culture, the percentage of T_{SCM} of the culture medium B group in the whole primary CD8⁺T cells, namely the induction efficiency, was up to 78.7%, and the induction efficiency of T_{SCM} of the culture medium D group was slightly lower (72.0%). The culture medium C group and E group, having TWS119 combined in the medium, achieved an induction efficiency of T_{SCM} of 87.0%. Furthermore, the inventors found that the inducing agents of the present invention could effectively induce the formation of T_{SCM} in the following concentration ranges: IL-7 (1 to 100 ng/mL), IL-15 (1 to 100 ng/mL), IL-21 (1 to 100 ng/mL), and TWS119 (1 to 50 µM).

Fig. 2 illustrates in-vitro induction and amplification effects of the culture media A to E according to the present invention on CD8⁺T_{SCM}.

Fig. 2A is a cell counting diagram illustrating CD8⁺T_{SCM} induced and amplified by using the culture media A to E according to the present invention. At Day 14 of culture, the induction and amplification effects of the culture medium A on CD8⁺T_{SCM} were the lowest, and the induction and amplification effects of the culture medium B group according to the present invention on CD8⁺T_{SCM} were significantly superior to those of the culture medium C group according to the present invention, while the induction and amplification effects of the culture medium D group according to the present invention on CD8⁺T_{SCM} were even better.

Fig. 2B illustrates induction and amplification multiples of CD8⁺T_{SCM} at Day 7 and Day 14 of culture by using the culture media A to E according to the present invention. After Day 14 of culture, the induction and amplification effects on CD8⁺T_{SCM} were as follows: the induction and amplification multiple of the culture medium A group according to the present invention was 800 folds, the induction and amplification multiple of the culture medium B group according to the present invention was 1162 folds, and the induction and amplification multiple of the culture medium D group according to the present invention was 5269 folds. The concentrations of IL-7, IL-15 and IL-21 for effectively amplifying CD8⁺T_{SCM} all ranged from 1 to 100 ng/mL.

### Example 2 In-vitro induced differentiation and amplification effects of the culture media B and D of the present invention on CD19 CAR-T_{SCM}

1) Pre-prepared and frozen healthy peripheral blood mononuclear cells (PBMCs) (Sinobay) were thawed and added into a 48-well cell culture plate (1×10⁶ PBMCs/well).
2) Subsequently, magnetic beads coupled to an anti-CD3 antibody and an anti-CD28 antibody (the number of cells: the number of magnetic beads=1: 1) and the culture medium B or D according to the present invention were added into the above cell culture plate. Three parallel wells were provided for each treatment.
3) After the cells were cultured for 1 day, a lentiviral vector LV-CD19CAR (Sinobay) was added into the above culture system, wherein multiplicity of infection (MOI) was equal to 5. After the cells were centrifugally infected at 1200 g and 32°C for 2 hours, a supernatant was discarded, the culture medium B or D was supplemented, and culturing was continued.

At Day 4 of cell culture, the whole of the above cell culture system was transferred to a 12-well plate, and the culture medium B or D was supplemented.

At Day 7 of cell culture, the magnetic beads coupled to an anti-CD3 antibody and an anti-CD28 antibody were removed, and the culture medium B or D was supplemented.

At Day 10 of cell culture, a fresh culture medium B or D was supplemented repeatedly as that at Day 7.

4) The cells were counted at Day 7 and Day 11 of cell culture respectively, and T_{SCM} (CD45RA, CCR7 and CD95) was detected by using a flow cytometry.

Experimental results are shown in Fig. 3.

Fig. 3 illustrates in-vitro induced differentiation and amplification effects of two T_{SCM} induction and amplification culture media B or D on CD19 CAR-T_{SCM}.

Fig. 3A illustrates a detection flow of the flow cytometry for CD19 CAR-T_{SCM} at Day 11 of culture and percentages of the corresponding cell subsets.

Fig. 3B illustrates a statistical result of the percentages of CD19 CAR-T_{SCM} in CD19 CAR-T cells at Day 7 and Day 11 of culture. The percentages of T_{SCM} (CD45RA⁺CCR7⁺CD95⁺) induced and differentiated by the culture medium B and the culture medium D reached 81.3% and 64.4% respectively in CD19 CAR-T cells cultured at Day 11, indicating that both media can effectively induce the differentiation of CD19 CAR-T_{SCM}.

Fig. 3C illustrates in-vitro induced differentiation and amplification effects of two T_{SCM} induction and amplification culture media on CD19 CAR-T_{SCM}.The experimental results showed that culture medium D could induce and amplify CD19 CAR-T_{SCM} more effectively.

### Example 3 In-vitro induced differentiation and amplification effects of the culture media B and D of the present invention on CD19 CAR-CD8⁺T_{SCM}

CD19 chimeric antigen receptor CD8⁺T cells (CD19 CAR-CD8⁺T cells) were prepared:
1) Pre-prepared and frozen healthy peripheral blood mononuclear cells (PBMCs) (Sinobay) were thawed, and then CD8⁺T cells were selected by a magnetic bead negative selection method, and were added into a 48-well cell culture plate (1×10⁶ CD8⁺T cells/well).
2) Subsequently, magnetic beads coupled to an anti-CD3 antibody and an anti-CD28 antibody (the number of cells: the number of magnetic beads=1: 1) and the culture medium B or D according to the present invention were added into the above cell culture plate. Three parallel wells were provided for each treatment.
3) After the cells were cultured for 1 day, a lentiviral vector LV-CD19CAR (Sinobay) was added into the above culture system, wherein multiplicity of infection (MOI) was equal to 5. The cells were centrifugally infected at 1200 g and 32°C for 2 hours. After centrifugation, the culturing was continued overnight, a supernatant was discarded, and the culture medium B or D according to the present invention was supplemented.

At Day 4 of cell culture, the whole of the above cell culture system was transferred to a 12-well plate, and the culture medium B or D according to the present invention was supplemented.

At Day 7 of cell culture, the magnetic beads coupled to an anti-CD3 antibody and an anti-CD28 antibody were removed, and the culture medium B or D according to the present invention was supplemented.

At Day 10 of cell culture, a fresh culture medium B or D according to the present invention was supplemented repeatedly as that at Day 7.

4) The cells were counted at Day 7 and Day 11 of cell culture respectively, and CD8⁺-T_{SCM} (CD45RA⁺CCR7⁺CD95⁺CD8⁺T cells) was detected by using a flow cytometry.

Experimental results are shown in Fig. 4.

Fig. 4 illustrates in-vitro induced differentiation and amplification effects of the two culture media B or D on CD19 CAR-CD8⁺T_{SCM}.

Fig. 4A illustrates a detection flow of the flow cytometry for CD19 CAR-CD8⁺T_{SCM} at Day 11 of culture and percentages of the corresponding cell subsets.

Fig. 4B illustrates a statistical result of the percentage of CD19 CAR-CD8⁺T_{SCM} in CD19 CAR-CD8⁺T cells. The percentages of T_{SCM} (CD45RA⁺CCR7⁺CD95⁺) induced and differentiated by the culture media B and D reached 93.2% and 82.5% respectively in CD19 CAR-CD8⁺T cells, indicating that the combination of two cytokines can effectively induce the differentiation of CD19 CAR-CD8⁺T_{SCM}.

Fig. 4C illustrates in-vitro induced differentiation and amplification effects of the two culture media B or D on CD19 CAR-CD8⁺T_{SCM}. The experimental results showed that the culture medium D could induce and amplify CD19 CAR-CD8⁺T_{SCM} more effectively.

### Example 4 In-vitro activation effects of CD19 CAR-CD8⁺ T cells cultured by the culture media B and D of the present invention

1) 1×10⁵ resting effector cell CD19 CAR-CD8⁺T cells taken from the CAR-T cells prepared in Example 3 and 1×10⁵ K562-CD19 target cells (Sinobay) were added into a 96-well U-bottom cell culture plate in a ratio of effector cells to target cells of 1: 1, the cells were centrifuged at 400 g for 1 min to promote the contact of the effector cells and the target cells;
2) After the effector cells and the target cells were co-cultured for 24 hours, the cells were centrifuged at 500 g for 5 minutes, a supernatant of the cultured cells was taken, and the content of cytokines IFN-γ in the supernatant was detected by ELISA.

Experimental results are shown in Fig. 5, CD19 CAR-CD8⁺T cells induced and amplified by using the two culture media B and D could effectively activate and secrete a large amount of functional effector molecules IFN-γ after contacting tumor target cells, and the culture medium D had a better activation effect. T cells in the untransduced control group (UTD) did not produce the effector molecules IFN-y.

### Example 5 In-vitro killing effects of CD19 CAR-CD8⁺T cells cultured by the culture media B and D according to the present invention

1) Prepared tumor cells K562-CD19 (Sinobay) were used as target cells, and a cell proliferation dye eFluor 450 (Thermo Fisher Scientific Co., Ltd.) and a cell membrane dye PKH26 (Sigma-Aldrich Trading Co., Ltd.) were used as labeling dyes respectively.

First, K562-CD19 cells were labeled with eFluor 450. The cells were washed twice with PBS or a serum-free RPMI1640 culture medium, and centrifuged at 500 g for 5 minutes to remove residual serum from the cells. The cells were resuspended in PBS (at least 500 µL of PBS) at a density of 2×10⁷/mL. An eFluor 450 stock solution was diluted to 10 µM by using PBS and then the diluted solution was mixed with the cell suspension in a ratio of 1: 1 (total volume 1 mL). The mixture was put into a water bath at 37°C, and reacted in dark for 10 minutes. The reaction was stopped by adding 200 µL of precooled FBS and the mixture was incubated on ice for 5 minutes. And then, the cells were washed with PBS for 3 times, and labeled with the cell membrane dye PKH26.

2) The above target cells were labeled with PKH26: refer to the manufacturer's instructions in a PKH26 kit for a labeling method. 2× single-cell suspension was prepared by using Diluent C provided in the kit, and the cell density was controlled at 2×10⁷/mL. Then, 2× PKH26 staining solution was prepared by using Diluent C, the 2× single-cell suspension was added into the 2× PKH26 staining solution in a ratio of 1: 1, and the mixture was reacted at room temperature for 5 min. Finally, FBS in an equal volume was added for reacting for 1 min. and staining was stopped. The cells were washed with a complete medium RPMI1640 (10% FBS) for 3 times for later use.

3) The number of the tumor target cells was fixed at 3×10⁴, the effector cells CD19 CAR-CD8⁺T cells prepared in Example 3 were added in a ratio of the effector cells to target cells of 2: 1, 4: 1, 8: 1 and 16: 1, respectively, the cells were co-incubated at 37°C for 4 hours, a cell suspension was collected, and the killing efficiency of the effector cells was detected by using a flow cytometry.

Experimental results are shown in Fig. 6, CD19 CAR-CD8⁺T cells induced and amplified by both the culture media B and D could kill the tumor target cells in a short time, and the effector cells cultured by the culture medium D had a better killing effect. T cells in the untransduced control group (UTD) hardly killed the tumor cells.

### Example 6 In-vivo anti-tumor effects of CD19 CAR-T_{SCM} cultured by the culture media B and E according to the present invention

1) 6-8-week-old female severe combined immunodeficiency (NSG) mice (Beijing Biocytogen Co., Ltd.) were used as models, and 5×10⁶ tumor cells (CD19⁺A549, Sinobay) were inoculated subcutaneously in the right flank of each mouse in an inoculation volume of 125 µL per mouse.
2) Six days after inoculation of the tumor cells, a visible tumor appeared under the skin on the right flank of each NSG mouse. Tumor mice were randomly divided into three groups, with 5 mice in each group. Three groups of mice were reinfused intravenously with different cells (all from Examples 2 and 3), which included the untransduced control group (UTD) T cells and CD19 CAR-T_{SCM} for treatment groups prepared by two methods (the culture medium B group and the culture medium D group). The cell reinfusion dose was 125 µL (10⁷ cells/mouse).
3) The tumor size was measured every 3 days after cell reinfusion by using a vernier caliper, and survival conditions of mice of each group were recorded.

A calculation formula for tumor volume was: tumor volume=(long diameter of tumor×wide diameter² of tumor)/2.

Experimental results are shown in Fig. 7.

Fig. 7A illustrates a schedule of in-vivo experiment of mice.

Fig. 7B and Fig. 7C are diagrams illustrating in-vivo anti-tumor effects of CD19 CAR-T_{SCM} cultured by the culture media B and E. As shown in the figures, the CD19 CAR-T_{SCM} induced and amplified by both the culture media could effectively inhibit the growth of tumor, wherein the tumor clearance rate of the culture medium B group was 80%, while the CD19 CAR-T_{SCM} prepared by the combined culture group of the culture medium D group had a better in-vivo anti-tumor effect, achieving rapid and efficient tumor clearance in vivo, with the tumor clearance rate as high as 100%. However, UTD T cells of the control group could not inhibit the growth of tumor.

Fig. 7D is a diagram of survival curves of mice in each group after CD19 CAR-T_{SCM} treatment. As shown in the figure, all the mice treated with CD19 CAR-T_{SCM} induced and amplified by the two culture media survived, while the mortality rate of the mice reinfused with UTD T cells of the control group was as high as 80%.

## Claims

1. A composition for inducing and/or amplifying T_{SCM} in vitro, the composition comprising inducing agents, the inducing agents comprising IL-7 and IL-21.

2. The composition of claim 1, wherein the inducing agents further comprise one or more of IL-3, IL-12, IL-15 and IL-18; preferably, the inducing agents further comprise IL-15.

3. The composition of claim 1 or claim 2, wherein the inducing agents further comprise a GSK-3β inhibitor; preferably, the GSK-3β inhibitor is TWS119; and
preferably, the inducing agents comprise IL-7, IL-21, IL-15 and a GSK-3β inhibitor.

4. The composition of any one of claims 1 to 3, wherein working concentrations of various inducing agents in the composition are as follows:
a working concentration of each of inducing agents, excluding a GSK-3β inhibitor, ranges from 1 to 100 ng/mL, and a working concentration of a GSK-3β inhibitor ranges from 1 to 50 µM;
preferably, the working concentrations of various inducing agents in the composition are as follows:
1 to 100 ng/mL of IL-7, and 1 to 100 ng/mL of IL-21;
preferably, the composition further comprises 1 to 100 ng/mL of IL-15; and
preferably, the composition further comprises 1 to 50 µM of a GSK-3β inhibitor.

5. A culture medium for inducing and/or amplifying T_{SCM} in vitro, wherein the culture medium comprises a T cell growth basal culture medium and a composition of any one of claims 1 to 4.

6. Use of a composition of any one of claims 1 to 4 or a culture medium of claim 5 for inducing and/or
amplifying T_{SCM} in vitro;
preferably, the T_{SCM} is PBMC, CD4⁺T cells, CD8⁺T cells or CD4⁻CD8⁻T cells; and
preferably, the T_{SCM} is CAR-T cells, TCR-T cells or tumor infiltrating lymphocytes.

7. A method for inducing and/or amplifying T_{SCM} in vitro, wherein the method comprises the step of inducing and/or amplifying cells by using a composition of any one of claims 1 to 4 and/or a culture medium of claim 5.

8. The method of claim 7, wherein the method comprises the steps of:
① separating PBMC, CD4⁺T cells, CD8⁺T cells or CD4⁻CD8⁻T cells;
② placing the cells obtained in step ① in a culture medium of claim 5 or in a T cell growth basal culture medium while adding a composition of any one of claims 1 to 4, adding a stimulant, and culturing for 6 to 7 days,
preferably, the stimulant is one or more of an anti-CD3 antibody, an anti-CD28 antibody/CD28 ligand, an anti-CD137 antibody/CD137 ligand, an anti-OX40 antibody/OX40 ligand, an anti-CD160 antibody/CD160 ligand, a TLR1 ligand, a TLR2 ligand, a TLR5 ligand, a TLR6 ligand, an RIG-I ligand, a chimeric antigen receptor target antigen, a PD-1 blocking antibody, a CTLA-4 blocking antibody, an LAG-3 blocking antibody, a Tm-3 blocking antibody, and a BTLA blocking antibody, and
more preferably, the stimulant is magnetic beads coupled to an anti-CD3 antibody and an anti-CD28 antibody; and
③ placing the cells obtained in step ② in the culture medium of claim 5 or in the T cell growth basal culture medium while adding the composition of any one of claims 1 to 4, and supplementing the culture medium of claim 5 or the T cell growth basal culture medium while adding the composition of any one of claims 1 to 4 every 2 to 4 days until the end of a cell culture cycle;
preferably, the method comprises the steps of:
① selecting CD8⁺T cells by a magnetic bead negative selection method;
② placing the cells obtained in step ① in the T cell growth basal culture medium, adding the composition of any one of claims 1 to 4, adding magnetic beads coupled to an anti-CD3 antibody and an anti-CD28 antibody, and culturing for 7 days; and
③ placing the cells obtained in step ② in the culture medium of claim 5 or in the T cell growth basal culture medium while adding the composition of any one of claims 1 to 4, and supplementing the fresh culture medium of claim 5 or the fresh T cell growth basal culture medium while adding the composition of any one of claims 1 to 4 every 3 days from Day 4 until the end of a cell culture cycle.

9. The method of claim 7, wherein the method is a method for inducing and/or amplifying CAR-T_{SCM} in vitro and comprises the steps of:
1) separating PBMC, CD4⁺T cells, CD8⁺T cells or CD4⁻CD8⁻T cells;
2) placing the cells obtained in step 1) in the culture medium of claim 5 or in the T cell growth basal culture medium while adding the composition of any one of claims 1 to 4, adding a stimulant, and culturing for 1 to 2 days,
preferably, the stimulant is one or more of an anti-CD3 antibody, an anti-CD28 antibody/CD28 ligand, an anti-CD137 antibody/CD137 ligand, an anti-OX40 antibody/OX40 ligand, an anti-CD160 antibody/CD160 ligand, a TLR1 ligand, a TLR2 ligand, a TLR5 ligand, a TLR6 ligand, an RIG-I ligand, a chimeric antigen receptor target antigen, a PD-1 blocking antibody, a CTLA-4 blocking antibody, an LAG-3 blocking antibody, a Tm-3 blocking antibody, and a BTLA blocking antibody, and
more preferably, the stimulant is magnetic beads coupled to an anti-CD3 antibody and an anti-CD28 antibody;
3) transfecting or infecting the cells obtained in step 2) with a vector carrying a chimeric antigen receptor; and
4) placing the cells obtained in step 3) in the culture medium of claim 5 or in the T cell growth basal culture medium while adding the composition of any one of claims 1 to 4, adding a stimulant, continuing culturing for 5 to 6 days, and then removing the stimulant; and supplementing the culture medium of claim 5 or the T cell growth basal culture medium while adding the composition of any one of claims 1 to 4 every 2 to 4 days from Day 4 until the end of a culture cycle of CAR-T cells;
preferably, the stimulant is one or more of an anti-CD3 antibody, an anti-CD28 antibody/CD28 ligand, an anti-CD137 antibody/CD137 ligand, an anti-OX40 antibody/OX40 ligand, an anti-CD160 antibody/CD160 ligand, a TLR1 ligand, a TLR2 ligand, a TLR5 ligand, a TLR6 ligand, an RIG-I ligand, a chimeric antigen receptor target antigen, a PD-1 blocking antibody, a CTLA-4 blocking antibody, an LAG-3 blocking antibody, a Tm-3 blocking antibody, and a BTLA blocking antibody, and
more preferably, the stimulant is magnetic beads coupled to an anti-CD3 antibody and an anti-CD28 antibody; and
preferably, the method comprises the steps of:
1) separating PBMC or CD8⁺T cells;
2) placing the cells obtained in step 1) in the culture medium of claim 5 or in the T cell growth basal culture medium while adding the composition of any one of claims 1 to 4, adding magnetic beads coupled to an anti-CD3 antibody and an anti-CD28 antibody, and culturing for 1 day;
3) adding a lentiviral vector LV-CAR carrying the chimeric antigen receptor into the cells obtained in step 2) in a proportion of multiplicity of infection (MOI) of 2-15, and performing centrifugal infection at 30 to 32°C for 2 h; and
4) after centrifugation is finished, culturing the cells obtained in step 3) overnight, placing the cells in the culture medium of claim 5 or in the T cell growth basal culture medium while adding the composition of any one of claims 1 to 4, adding magnetic beads coupled to an anti-CD3 antibody and an anti-CD28 antibody, continuing culturing for 5 to 6 days, and then removing the magnetic beads coupled to an anti-CD3 antibody and an anti-CD28 antibody; and supplementing the culture medium of claim 5 or the T cell growth basal culture medium while adding the composition of any one of claims 1 to 4 every 3 days from Day 4, and continuing culturing until the end of a culture cycle of CAR-T cells.

10. The method of claim 7, wherein the method is a method for inducing and/or amplifying TCR-T_{SCM} in vitro and comprises the steps of:
1) separating PBMC, CD4⁺T cells, or CD8⁺T cells;
2) placing the cells obtained in step 1) in the culture medium of claim 5 or in the T cell growth basal culture medium while adding the composition of any one of claims 1 to 4, adding a stimulant, and culturing for 1 to 2 days,
preferably, the stimulant is one or more of an anti-CD3 antibody, an anti-CD28 antibody/CD28 ligand, an anti-CD137 antibody/CD137 ligand, an anti-OX40 antibody/OX40 ligand, an anti-CD160 antibody/CD160 ligand, a TLR1 ligand, a TLR2 ligand, a TLR5 ligand, a TLR6 ligand, an RIG-I ligand, a chimeric antigen receptor target antigen, a PD-1 blocking antibody, a CTLA-4 blocking antibody, an LAG-3 blocking antibody, a Tm-3 blocking antibody, and a BTLA blocking antibody, and
more preferably, the stimulant is magnetic beads coupled to an anti-CD3 antibody and an anti-CD28 antibody; and
3) transfecting or infecting the cells obtained in step 2) with a vector carrying a T cell receptor; and
4) placing the cells obtained in step 3) in the culture medium of claim 5 or in the T cell growth basal culture medium while adding the composition of any one of claims 1 to 4, adding a stimulant, continuing culturing for 5 to 6 days, and then removing the stimulant; and supplementing the culture medium of claim 5 or the T cell growth basal culture medium while adding the composition of any one of claims 1 to 4 every 2 to 4 days from Day 4 until the end of a culture cycle of TCR-T cells,
preferably, the stimulant is one or more of an anti-CD3 antibody, an anti-CD28 antibody/CD28 ligand, an anti-CD137 antibody/CD137 ligand, an anti-OX40 antibody/OX40 ligand, an anti-CD160 antibody/CD160 ligand, a TLR1 ligand, a TLR2 ligand, a TLR5 ligand, a TLR6 ligand, an RIG-I ligand, a chimeric antigen receptor target antigen, a PD-1 blocking antibody, a CTLA-4 blocking antibody, an LAG-3 blocking antibody, a Tm-3 blocking antibody, and a BTLA blocking antibody, and
more preferably, the stimulant is magnetic beads coupled to an anti-CD3 antibody and an anti-CD28 antibody; and
preferably, the method comprises the steps of:
1) separating PBMC, CD4⁺T cells, or CD8⁺T cells;
2) placing the cells obtained in step 1) in the culture medium of claim 5 or in the T cell growth basal culture medium while adding the composition of any one of claims 1 to 4, adding magnetic beads coupled to an anti-CD3 antibody and an anti-CD28 antibody, and culturing for 1 day;
3) adding a lentiviral vector LV-TCR carrying a chimeric antigen receptor into the cells obtained in step 2) in a proportion of multiplicity of infection (MOI) of 2-15, and performing centrifugal infection at 30 to 32°C for 2 h; and
4) after centrifugation is finished, culturing the cells obtained in step 3) overnight, placing the cells in the culture medium of claim 5 or in the T cell growth basal culture medium while adding the composition of any one of claims 1 to 4, adding magnetic beads coupled to an anti-CD3 antibody and an anti-CD28 antibody, continuing culturing for 5 to 6 days, and then removing the magnetic beads coupled to an anti-CD3 antibody and an anti-CD28 antibody; and supplementing the culture medium of claim 5 or the T cell growth basal culture medium while adding the composition of any one of claims 1 to 4 every 3 days from Day 4, and continuing culturing until the end of a culture cycle of TCR-T cells.

11. T_{SCM} prepared by a method of any one of claims 7 to 10.

12. Use of a T_{SCM} of claim 11 in the manufacture of a medicament for treating tumors.

13. A method for cellular immunotherapeutically treating tumors, wherein the method comprises using a T_{SCM} of claim 11.
